(19) Europäisches Patentamt
European Patent Office
Office européen des brevets

(11) Publication number: **0 317 780 B1**

(12) **EUROPEAN PATENT SPECIFICATION**

(45) Date of publication of patent specification: **20.05.92** (51) Int. Cl.5: **A61K 31/44**, A61K 47/34

(21) Application number: **88117844.6**

(22) Date of filing: **26.10.88**

(54) **Sustained (controlled) release delivery system for substituted dihydropyridine calcium channel blockers.**

(30) Priority: **25.11.87 US 125440**

(43) Date of publication of application:
**31.05.89 Bulletin 89/22**

(45) Publication of the grant of the patent:
**20.05.92 Bulletin 92/21**

(84) Designated Contracting States:
**AT BE CH DE ES FR GB GR IT LI NL SE**

(56) References cited:
**WO-A-83/02230**
**BE-A- 897 821**
**FR-A- 2 081 436**
**GB-A- 2 140 687**

(73) Proprietor: **AMERICAN CYANAMID COMPANY**
**1937 West Main Street P.O. Box 60**
**Stamford Connecticut 06904-0060(US)**

(72) Inventor: **Banesan, Madurai Gurusamy**
**59 Lonergan Drive**
**Suffern New York 10901(US)**
Inventor: **Desal, Narendra Ragjunathji**
**4 Rolf Drive**
**Danbury Connecticut 06811(US)**
Inventor: **Maier, Gary Arthur**
**27 Gregory Drive**
**Goshen New York 10924(US)**
Inventor: **Kulkarni, Prakash Shriram**
**13 Lord Sterling Drive**
**Morris New Jersey 07054(US)**

(74) Representative: **Wächtershäuser, Günter, Dr.**
**Tal 29**
**W-8000 München 2(DE)**

**EP 0 317 780 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with a formulation that provides for the sustained release of 1,4-dihydropyridines after oral administration.

Sustained or controlled release formulations that provide for continued release of orally administered pharmaceuticals. In the prior art these formulations have been based on coatings that are gradually eroded or dissolved. In addition, it is known to use discrete polymeric units that have been impregnated with a drug that will be slowly leached from the polymeric unit by the action of gastro intestinal fluids. In order to prepare sustained or controlled release formulations of 1,4-dihydropyridine, it is important that the drug is adequately soluble in water or in a pharmaceutically acceptable solvent in order to achieve therapeutic blood levels. It is also important that the solubility characteristics be substantially unaffected by environmental conditions (heat, light, moisture) or by the manufacturing process that is employed to make the sustained or controlled release composition. PCT application WO 83/2230 (CA 102(8) 154832) discloses dry capsules that were prepared with nifedipine, sucrose stearate, Pluronic F 68®, lactose and methyl cellulose. CA 99(4): 27904d discloses a solidified melt of griseofulvin-Pluronic F® 68 that shows increased bioavailability as compared to micronized griseofulvin.

The applicants have discovered that the solubility of the poorly soluble 1,4-dihydropyridines such as nilvadipine and nifedipine may be modified by forming a complex of said 1,4-dihydropyridines with a polyoxypropylene-polyoxyethylene block copolymer. These complexes have sufficient water solubility for use in the preparation of a sustained or controlled release pharmaceutical composition.

These complexes are stable over a wide temperature range and exhibit higher bioabsorption than presently available systems.

There are provided complexes which comprise of a 1,4-dihydropyridine with one or more polyoxypropylene-polyoxyethylene block copolymers, as defined in claim 1.

Also provided by the invention are sustained release compositions of 1,4-dihydropyridine and one or more polyoxypropylene-polyoxyethylene block copolymers in combination with a material that forms a low viscosity transient gel such as a water soluble cellulose derivative.

The invention also provides a novel quick release and sustained release formulations of 1,4-dihydropyridines-complexes. By selection and blending of the water soluble cellulose derivative, it is possible to prepare dosage formulation that deliver the drugs in a bioavailable form over periods of from 10 minutes to 24 hours.

FIG. 1 is a diagram that depicts the behavior of nilvadipine-polyoxyethylene-polyoxpropylene complex.

FIG. 2 is a graph which compares the dissolution profiles of nifedipine formulations in simulated intestinal fluid.

FIG. 3 is a graph which compares the dissolution profiles of bilayer tablets of nilvadipine formulations in simulated intestinal fluid dissolution medium containing 0.4% Tween® 20.

FIG. 4 is a graph which depicts the effect of 14 days of 75% relative humidity on the dissolution profiles of SR tablets containing 10 mg of nifedipine in simulated intestinal fluid.

FIG. 5 is a graph which composes the dissolution profiles of 10 mg QR and SR formulation of nifedipine simulated intestinal fluid at 37° C.

FIG. 6 is a graph that gives the shows the release rates of quick release and sustained release tablets of nilvadipine-polyoxyethylene-polyoxypropylene complex.

FIG. 7 is a graph that illustrates the release rate of a 20 mg bilayer tablet of QR and SR nilvadipine complex.

## DETAILED DESCRIPTION OF THE INVENTION

Suitable 1,4-dihydropyridines that may be utilized in the practice of the invention include nilvidapine or its (-) or (+) enantiomers, nitredipine, nisoldipine, niludipine, nicardipine, nifedipine, and nimodipine. These types of compounds are described in U.S. 4,264,611; U.S. 4,510,150; U.S. 4,338,322; U.S. 4,284,634; U.S. 4,504,476; and U.S. 4,520,112.

Preferred 1,4-dihydropyridines include those of the general formula:

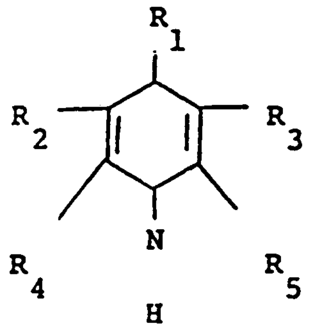

or a resolved enantiomer thereof, wherein

$R_1$ is aryl,

$R_2$ and $R_3$ are each, same or different, ester groups or carboxy groups, and

$R_4$ and $R_5$ are each hydrogen, cyano, lower alkyl, or substituted lower alkyl in which the substituent is cyano, hydroxy, acyloxy, hydroxyimino, hydrazono, lower alkoxyimino, hydroxy(lower)alkylimino, N′ -or N′,N′-di(lower)alkylamino(lower)alkylimino, hydrazino, hydroxy(lower)alkylamino, N′-or N′,N′-di(lower)-alkylamino(lower)alkylamino, a 5 or 6-membered saturated N-containing heterocyclic-1-yl which may have hydroxy, lower alkyl or hydroxy(lower)alkyl, or oxo wherein the thus formed carbonyl may be protected with suitable protecting group; provided that, when one of $R_4$ and $R_5$ is hydrogen or lower alkyl, the other is always cyano or said substituted lower alkyl, and when $R_4$ and $R_5$ are not hydrogen or lower alkyl, both of them are a group selected from cyano and said substituted lower alkyl,

or $R_4$ is hydrogen or lower alkyl and $R_3$ and $R_5$ are combined to form a group of the formula:

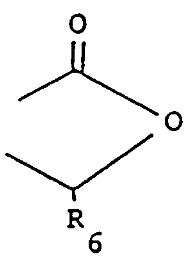,

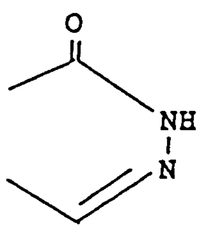,

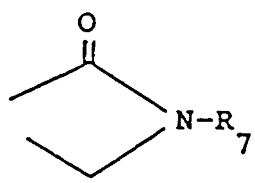 or

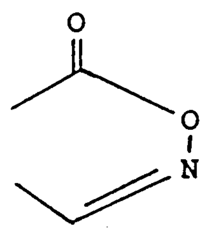

wherein $R_6$ is hydrogen or methyl and $R_7$ is 2-(N,N-diethylamino)ethyl or 2-hydroxyethyl.

The terms used in the definitions of the symbols of the general formulae given in this specification and claims are explained as follows:

The term "lower" used in connection with an alkylene, alkyl and alkenyl is intended to mean the one having 1 or 2 to 8 carbon atoms.

The aryl and aryl moieties may be phenyl, naphthyl, xylyl, tolyl, mesityl, and cumenyl, which may have one or more suitable substituent(s). Preferred examples of the suitable substituent(s) are halogen, nitro, hydroxy, halo(lower)-alkyl, lower alkoxy, lower alkenyloxy, cyano, lower alkoxycarbonyl or lower alkylsulfamoyl. The halogen or halo moieties are fluorine, chlorine, bromine or iodine.

Lower alkylene moieties may have a straight or branched and saturated bivalent hydrocarbon chain such as methylene, ethylene, methylmethylene, trimethylene, propylene or tetramethylene.

Lower alkyl and lower alkyl moieties may have a straight or branched and saturated hydrocarbon chain such as methyl, ethyl, propyl, isopropyl, butyl, isobutyl, t-butyl, pentyl, neo-pentyl, hexyl, heptyl or octyl.

Lower alkoxy and lower alkoxy moieties may be methoxy, ethoxy, propoxy, isopropoxy, butoxy, t-butoxy and pentyloxy.

Halo(lower)alkyl moieties may be mono-halo(lower)alkyl such as chloromethyl, bromomethyl or chloropropyl; di-halo(lower alkyl such as 1,2-dichloroethyl, 1,2-dibromoethyl or 2,2-dichloroethyl; and tri-halo(lower)alkyl such as tri-fluoromethyl or 1,2,2,-trichloroethyl.

Lower alkenyl and lower alkenyl moieties may be ones having a straight or branched hydrocarbon chain which contains one or more double bond(s), such as vinyl, allyl, butenyl, butanedienyl or penta-2,4-dienyl.

Acyl and acyl moieties may be lower alkanoyl such as formyl, acetyl, propionyl, butyryl, isobutyryl, valeryl, isovaleryl, pivaloyl; substituted lower alkanoyl, for example, carboxy(lower)-alkanoyl, esterified carboxy(lower)alkanoyl such as lower alkoxycarbonyl(lower)alkanoyl, N-or N,N-di-substituted amino(lower)-alkanoyl such as N-or N,N di-(lower)alkylamino(lower)alkanoyl (e.g. N-methyl-(or N,N-diethyl) aminoacetyl, 1(or2)-[N-ethyl(or N,N-diethyl)amino]proprionyl or 1 (or 2)-[N-methyl N-ethylamino]propionyl) or N-lower alkyl-N-ar(lower)alkylamino(lower)alkanoyl (e.g. 1-(or 2)-[N-methyl-N-benzylamino]propionyl) or aryloxy-(lower)alkanoylsuch as phenoxyacetyl, tolyloxyacetyl, 2(or 3 or 4)-chlorophenoxyacetyl, 2-[2(or 3 or 4)-chlorophenoxy]propionyl, 2(or 3 or 4)-nitrophenoxyacetyl or 2(or 3 or 4)-methoxyphenoxyacetyl); aroyl such as benzoyl, naphthoyl or toluoyl.

Lower alkoxycarbonyl moieties may be methoxycarbonyl, ethoxycarbonyl, propoxycarbonyl, isopropoxycarbonyl, butoxycarbonyl and t-butoxycarbonyl.

Lower alkylsulfamoyl moieties may be methylsulfamoyl, ethylsulfamoyl, propylsulfamoyl, isopropylsulfamoyl, butylsulfamoyl and pentylsulfamoyl.

A heterocyclic group designated $R_1$ may be an aromatic heterocyclic group containing one or more hetero atom(s) selected from a nitrogen atom, a sulfur atom and an oxygen atom, for example, thienyl, furyl, pyrrolyl, thiazolyl, thiadiazolyl, tetrazolyl, pyridyl, pyrimidinyl, quinolyl, isoquinolyl, benzothienyl, indolyl or purinyl.

Esterifed carboxy groups designated $R_2$ and $R_3$ may be lower alkoxycarbonyl such as methoxycarbonyl, ethoxycarbonyl, propoxycarbonyl, isopropoxycarbonyl, butoxycarbonyl, t-butoxycarbonyl; halo(lower)-alkoxycarbonyl such as the haloanalogues of the above-mentioned lower alkoxycarbonyl (e.g., 2-bromoethoxycarbonyl, 2-chloroethoxycarbonyl, 2(or 3)-chloropropoxycarbonyl, 2 (or 3)-bromopropoxycarbonyl, 2,2-dichloroethoxy-carbonyl or 2,2,2-trichroloethoxycarbonyl); hydroxy(lower)alkoxycarbonyl such as 2-hydroxyethoxycarbonyl or 2(or 3)hydroxypropoxycarbonyl; lower alkoxy(lower)alkoxycarbonyl such as 2-methoxyethoxycarbonyl, 2-ethoxyethoxycarbonyl or 2(or 3)-methoxy(or ethoxy)-propoxycarbonyl; aryloxycarbonyl such as phenoxycarbonyl, tolyloxycarbonyl, xylyloxycarbonyl or p-chlorophenoxycarbonyl; ar-(lower)alkoxycarbonyl such as benzyloxycarbonyl, p-bromobenzyloxycarbonyl, O-methoxybenzyl-oxycarbonyl or phenethyloxycarbonyl; ar(lower)alkoxy(lower)alkoxycarbonyl such as 2-(benzyloxyl)-ethoxycarbonylor 2(or 3)-(benzyloxy)propoxycarbonyl; aryloxy(lower)alkoxycarbonyl such as 2-(phenoxy)-ethoxycarbonyl or 2(or 3)-(phenoxy)propoxycarbonyl; Nor N,N-(di)-substituted amino(lower)alkoxycarbonyl such as Nor N,N-(di)-(lower)alkylamino(lower) alkoxycarbonyl (e.g., 1(or 2)-[N-methyl(or N,N-dimethyl)-amino]ethoxycarbonyl, 1(or2)-[N-ethyl(or N,N-diethyl)amino]ethoxycarbonyl, or 1(or 2)-N-methyl-N-ethylamino)ethoxycarbonyl or lower alkyl-N-ar(lower)alkylamino(lower)alkoxycarbonyl (e.g. 2-(N-methyl-N-benzylamino)ethoxycarbonyl), and further $R_2$ and $R_3$ may be same or different.

Lower alkyl substituted with oxo includes lower alkanoyl such as formyl, acetyl, propionyl, butyryl, isobutyryl, valeryl, isovaleryl or pivaloyl and lower alkanoyl(lower)alkyl such as formylmethyl, acetonyl, 2-formylethyl, 3-formylpropyl or butyrylmethyl. The carbonyl group thereof may be protected with suitable protecting group, and thus protected carbonyl group in this invention means a group given by protecting the carbonyl with conventionally employed protecting group for a carbonyl. Suitable examples of such protected carbonyl groups are acetal, cyclic-acetal, thioacetal, cyclic-thioacetal, cyclicmonothioacetal or acylal types of group. Examples of these lower alkyl groups containing such protected carbonyl group are gem-di-(lower)alkoxy(lower)alkyl (e.g., dimethoxymethyl, 1,1-dimethoxyethyl, diethoxymethyl, dipropoxymethyl, 2,2-diethoxyethyl or 2,2-diethoxypropyl; gem-lower alkylenedioxy(lower)alkyl (e.g. 1,3-dioxolan-2-yl, 2 methyl-1,3-dioxolan-2-yl, 4-methyl-1,3-dioxolan-2-yl, 4,5-dimethyl-1,3-dioxolan-2-yl, 1,3-dioxan-2-yl, 2-methyl-1,3-dioxan-2-yl, 1,3-dioxolan-2-yl-methyl, 2-methyl-1,3-dioxolan-2-yl-methyl or 3-(1,3-dioxolan-2-yl)propyl); gem-di-(lower)alkylthio(lower)-alkyl (e.g., dimethylthiomethyl, 1,1-dimethylthioethyl, diethylthiomethyl or 2,2-diethylthioethyl); gem-lower alkylenedithio(lower)alkyl (e.g. 1,3-dithiolan-2-yl, 2-methyl-1,3-dithiolan-2-yl, 4-methyl-1,3-dithiolan-2-yl, 4,5-dimethyl-1,3-dithiolan-2-yl, 1,3-dithian-2-yl, 2-methyl-1,3-dithian-2-yl, 1,3-dithiolan-2-yl-methyl, 2-methyl-1,3-dithiolan-2-ylmethyl or 3-(1,3-dithiolan-2yl)propyl);and gem-di(lower)-alkanoyloxy(lower)alkyl (e.g., diacetoxymethyl, 1,1-diacetoxyethyl, dipropionyloxymethyl or 2,2-dipropionyloxyethyl); 5 or 6-membered saturated 1-oxa-3-thioheterocyclic-1-yl-(lower)alkyl (e.g., 1,3-oxathiolan-2-yl, 2-methyl-1,3-oxathiolan-2-yl, 4-methyl-1,3-oxathiolan-2-yl, 4,5-dimethyl-1,3-oxathiolan-2-yl, 1,3-oxothian-2-yl, 2-methyl-1,3-oxothian-2-yl, 1,3-oxathiolan-2-ylmethyl, 2-methyl-1,3-oxathiolan-2-ylmethyl or 3-(1,3-oxathiolan-2-yl)propyl).

A 5 or 6-membered saturated N-containing heterocyclic-1-yl) group may be one which may contain additional one or more hetero atom(s) selected from nitrogen, sulfur and oxygen atoms such as pyrrolidin-1-yl, piperidino, imidazolidin-1-yl, morpholino or thiomorpholino, and it may be optionally substituted with hydroxy, lower alkyl or hydroxy(lower)alkyl such as hydroxymethyl, 2-hydroxyethyl, 2-hydroxypropyl or 3-hydroxypropyl.

The other terms of each lower alkoxyimino, N′- or N′,N′-di-(lower)alkylamino(lower)alkylimino, hydroxy-(lower)alkylamino, N′- or N′,N′-di(lower)alkylamino(lower)alkylamino and hydroxy(lower)alkylamino will be clearly defined by applying optionally the above given exemplifications of the terms to them.

Included in this general formula is 2-cyano-1,4-dihyro-6-methyl-4-(3-nitrophenyl)-3,5-pyridinedicarboxylic acid, 3-methyl-5-(1-methylethyl)ester.

All of the dihydropyridine derivatives which contain an asymmetric carbon atom when totally synthesized comprise racemates consisting of two enantiomers designated (+) and (-) as a consequence of the presence of the asymmetric carbon, for example, at the 4 position. These enantiomers may be resolved and isolated in ways known to those skilled in this art, such as, for example, by chiral chromatography of the racemate or by other means.

The polyoxypropylene-polyoxyethylene block copolymers are known materials which are commercially available or may be prepared by those skilled in the art. These block copolymers are prepared by the sequential addition of propylene oxide and ethylene oxide to propylene glycol and have the general structure:

$$HO(CH_2CH_2O)_a\ (CH_2\overset{\overset{CH_3}{|}}{C}HO)_b\ (CH_2CH_2O)_c H$$

when a, b and c are selected to provide a weight average molecular weight from 1000 to or over 16,000. These block copolymers may contain from 10 to 80% by weight of oxyethylene units. Preferred block copolymers are commercially available as Pluronic® F-127 which has a melting point of 56°C, a weight average molecular weight of 12,000; and a content of 70% oxyethylene units, and Pluronic® F-68 which has a melting point of 47°C; a weight average molecular weight of 6,000 and a content of 80% oxyethylene groups.

The complex may be formed by contacting the 1,4 dihydropyridine with the block copolymer in the presence of a suitable solvent. Suitable solvents include absolute ethanol, methylene chloride, isopropanol, propanol, n-butanol and chloroform. The procedure may be carried at from 25-90°C and under atmospheric pressure or superatomospheric pressure. Generally the ratios of 1,4-dihydropyridine: polyoxypropylene-polyoxyethylene block copolymers may be from 1:1 to 1:10 (w/w) preferably from about 1:5 w/w and an amount of the 1,4-dihydropyridine may be employed that will provide a concentration of 1-100 µl/mg and preferably 6-15 µl/mg of solvent to 1,4-dihydropyridine. Any suitable reactor may be utilized. although a glass or stainless steel reactor is preferred. The complex may be recovered from the reaction mixture by vacuum evaporation or by other suitable procedures. In the alternative the reaction mixture may be used directly for the preparation of a sustained release dosage formulation.

As shown in FIG. 1, when the complex of nilvadipine and polyoxypropylenepolyoxyethylene comes into contact with water in a physiological environment, it is converted into a solution $N_s$ that is subjected to two competing phenomena: (a) absorption through the biological membrane such as the gastrointestinal mucosa in the $N_m$ state or (b) precipitation in the lowest energy equilibrium solid state ($N_p$). The solid equilibrium state $N_p$ form is not bioabsorbed and the extent of the conversion of $N_s$ to $N_p$ determines the bioavailability of nilvadipine or the particular 1,4-dihydropyridine that is employed.

The sustained release formulation, which is also the subject of this invention, is designed for 1,4-dihydropyridine compounds having the following properties:

(a) poor water solubility which requires conversion to highly water soluble complex forms or polymorphs for sustained release delivery;

(b) water soluble forms or polymorphs which convert to equilibrium states having reduced water solubility and as a result exhibit little or no bioabsorption.

The conversion to different forms is due to water or any constituent in the physiological environment that competes for the ligand in the complex causing displacement of the drug.

The sustained release formulations may comprise:

(a) an erodible homogeneous matrix in a tablet, caplet or other geometrical shaped dosage form that when contacted with water will release the active components by causing successive layers of the dosage form to sequentially hydrate or solvate. As the successive layers are hydrated or solvated, the drug is released only from that region of the dosage form. The successive "onion like" dry layers do not have any channels or tortuous paths that could potentially contribute to the formation of microenvironments of a saturated solution

of the drug. In sustained release dosage forms of 1,4-dihydropyridine, it is advantageous to avoid the formation of channels in the matrix because if such channels are blocked the drug will precipitate and as a result will not be bioavailable.

It is believed that the interaction of polyoxypropylene-polyoxyethylene block copolymers with 1,4-dihydropyridines will prevent immediate hydration of the polymer-drug complex, however, the inventors do not wish to be bound by any theoretical basis on which the invention operates. The selection of a suitable block-copolymer will permit the formation of a relatively stable complex that will exhibit a proper degree of resistance to attack by water. FIG. 4 sets forth the effect of 75% relative humidity on the dissolution in water of a 1:5 complex of nifedipine and Pluronic® F-127. As noted hereinabove, generally from 1:1 to 1:10 weight percent of drug to block copolymer may be used. In the case of nilvadipine and Pluronic® F-127 the ratio of 1:5 weight percent of drug to Pluronic® F-127 was found to be optimum.

It is preferred to react the drug in a non-chlorinated solvent such as a straight or branched chain lower alkanol having 1-7 carbon atoms such as methanol, ethanol, n-propanol and n-butanol although other non-reactive solvents may be employed.

A pharmaceutical excipient which is a water soluble cellulose polymeric derivative that does not cause disintegration may be utilized in the preparation of the sustained release dosage forms. The proportion of excipient to complex will be varied so that the finished dosage form will have the desired pharmacologically effective amount of the drug. These excipients should be selected so that the molecular weight is sufficient that on contact with water they will generate a low viscosity transient gel by swelling which prevents nucleation of the drug in the solution state. Suitable celluose polymeric materials include hydroxypropyl cellulose (L-HPC) and hydroxy propylmethyl cellulose (HPMC).

Sustained release formulations of 1,4-dihydropyridine complex may be prepared with hydroxypropyl methyl cellulose having a molecular weight which provides a viscosity of 9-30 mPa.s at 20° C as a 2% w/w aqueous solution in water. A quick release formulation may be prepared with 3-8 mPa.s (2% $H_2O$-20° C) hydroxypropyl methyl cellulose and the 1,4-dihydropyridine complex.

In the preparation of sustained release formulations of complexed 1,4-dihydropyridine, it has been found that the selection and blending of water soluble cellulose derivatives having different molecular weights will provide formulations that deliver complexed 1,4-dihydropyridines for oral absorption at different rates. For example, the use of a hydroxypropylmethyl-cellulose polymer having a molecular weight that exhibits a viscosity of 6 mPa.s at 20° C as a 2% w/w solution in water with a complex of nilvadipine-polyoxypropylenepolyoxyethylene copolymer will have a quick release rate.

A quick release formulation may be prepared with a ratio of 0.15 to 1 to 0.5 to 1 of 6 mPa.s hydroxypropylmethyl cellulose to nilvadipine-polyoxypropylene-polyoxyethylene block copolymer complex. A 70:30 to 30:70 blend of a 6 mPa.s and 15 mPa.s (2% w/w solution in water at 20° C) hydroxypropylmethyl cellulose polymer with from 2:1 to 4:1 of nilvadipine-nilvadipine-polyoxypropylene-polyoxyethylene block copolymer complex may be used to prepare a sustained release formulation. Sustained release formulations of other 1,4-dihydropyridine may also be prepared using other water soluble cellulose derivatives which exhibit the desired delivery rates.

The addition of moisture free carbohydrate excipients preferably materials such as lactose, in amounts of from 10-50% by weight of the formulation facilities the slow controlled erosion of the low viscosity transient gel formed by the hydration of the matrix of the complex of the drug block copolymer and the low viscosity excipient which is a water soluble cellulose derivative.

As used herein, the term quick release means a dosage formulation that delivers 100% of the drug in 10 to 30 minutes. The term sustained release is used to include a dosage formulation that delivers approximately a constant fraction of the total dose over 3 to 24 or preferably 12 to 24 hours.

## EXAMPLE 1

### Complex Formation

In a clean stainless steel beaker is placed 1.56kg of ethanol U.S.P. (95%) which is warmed to about 45° C. While stirring continuously at that temperature, 1.0kg of Pluronic® F-127 is added to the warm ethanol and the mixture is stirred until the Pluronic® F-127 completely dissolves and 0.2kg of nilvadipine is added in divided amount so that the ratio of nilvadipine to Pluronic® F 127 is 1:5 w/w. A clear deep orange colored solution was obtained that contained the nilvadipine - Pluronic® F-127 complex.

EXAMPLE 2

Granulation

To 1.5kg of cps HPMC and 1.5kg of 15 mPa.s HPMC in a Hobart mixer is added the warm (40-42° C) nilvadipine - Pluronic® F-127 complex ethanol solution of Example 1. The Hobart mixer is set at medium speed and mixing is carried out for 5 minutes when 0.75 kg of anhydrous lactose is added in small increments. After the lactose is added, mixing is continued at medium speed for 5 10 minutes. A wet granulation is obtained which is vaccum dried in trays under controlled conditions.

The granulate is evenly spread in lined trays and placed in a vacuum oven at 40-45° C. The contents are vacuum dried over a 16-24 hour period until a moisture level of 1.0% is achieved. Rapid drying and temperatures over 45° C are to be avoided to prevent the conversion of the drug-polymer complex into an insoluble form of the drug by crystallization.

The dried granules are sieved through a 700 µm (25 mesh) U.S.P. sieve, placed in a V-blender and combined with 0.050kg of magnesium stearate and mixed for 5-10 minutes until a homogeneous mixture is obtained. At this point the granules are ready for tableting as the sustained release layer of a bilayer sustained release tablet.

The quick release portion is prepared from a nilvadipine complex prepared from 0.15 kg of nilvadipine; 0.75 kg of Pluronic® F 127 and 1.17 kg of ethanol U.S.P. (95%).

A Hobart mixer is loaded with 0.25kg of 6 mPa.s HPMC with the mixing speed set on low speed. A warm (40-45° C) alcoholic solution of the complex is added in small increments to assure that the contents are uniformly mixed. The Hobart mixer is set at medium speed for 5 minutes and 3.80 kg of anhydrous lactose is added in small incremental amounts. Mixing is continued for 5-10 minutes at medium speed. The granulation is then vacuum dried and sieved through a 700 µm (25 mesh) U.S.P. screen, placed in a V-blender and mixed with 0.050kg. of magnesium stearate and mixed for 5-10 minutes until a homogenous mixture is obtained.

Tablet Preparation

A bilayer tablet press that had been separately provided with quick release and sustained release granulation. The tooling size is 10 mm (13/32") (FFBE) for 12 mg and 16 mg size tablets and 11 mm (7/16") (FFBE) for 20mg size tablets.

The following individual layer weights were used:

| | Nilvadipine | | Granulate | |
|---|---|---|---|---|
| | Q.R. | S.R. | Q.R. | S.R. |
| 20 mg | 8 mg | 12 mg | 279 mg | 312 mg |
| 16 mg | 8 mg | 8 mg | 279 mg | 208 mg |
| 12 mg | 8 mg | 4 mg | 279 mg | 104 mg |

At different times in the tablet compression process, the weights of the individual layers were checked and adjusted, if necessary, similarly for each dose strength of the tablet measurements for friability, thickness and hardness were taken at different intervals during the entire run.

EXAMPLE 3

By using procedures that are analogous to Example I, a nifedipine-complex is prepared and tableted with each tablet containing 10 mg of nifedipine. Tablets containing 10 mg of nifedipine in a physical mixture with Pluronic® F-127 are compared with tablets containing 10 mg of nifedipine without Pluronic® F-127 and with tablets containing 10 mg of nifedipine complexed with Pluronic® F-127 as shown in FIG. 2. The physical mixture and the complex contained the following:

| | Physical Mix and Complex %(w) | Control %(w) |
|---|---|---|
| Nifedipine | 4 | 4 |
| Pluronic® F-127 | 16 | - |
| HPMC 15 mPa.s | 75 | 75 |
| Lactose | 4 | 20 |
| Mg stearate | 1 | 1 |

The complex was prepared by dissolving the nifedipine and the Pluronic® F-127 in ethanol at about 40 ° C and thereafter dispersing the ethanol solution on the lactose according to the general procedure of Example 2. The physical mixture is made by admixing all excipients in the absence of ethanol. The dissolution profile for 16 mg bilayer nilvadipine tablets is shown in FIG. 3 wherein the nilvadipine complex with HPMC and the physical mixture of nilvadipine had the following formulation:

| | Physical Mix and Sustained Release Layer (8mg) | Control (SR) |
|---|---|---|
| Nilvadipine | 4% | 4% |
| Pluronic® F-127 | 20% | - |
| HPMC 6 mPa.s | 30% | 30% |
| HPHC 15 mPa.s | 30% | 30% |
| Mg stearate | 1% | 1% |
| Lactose | 15% | 35% |

| | Quick Release (8mg) | Control (QR) |
|---|---|---|
| Nilvadipine | 3% | 3% |
| Pluronic® F-127 | 15% | - |
| HPMC 6 mPa.s | 5% | 5% |
| Lactose | 76% | 91% |
| Mg Stearate | 1% | 1% |

The tablets were prepared according to the general procedures set forth hereinabove.

The data set forth in FIG. 2 and FIG. 3 establish the effectiveness of the complexed 1,4-dihydropyridines as a dosage formulation.

EXAMPLE 4

FIG. 5 is sets forth the release rate for one quick release formulation of nifedipine-Pluronic® F-127 complex and two sustained release formulations of the same complex. It can be seen that by varying the type or by blending different types of hydroxypropylmethyl cellulose (HPMC), different release rates may be obtained.

The 6mPa.s, 6mPa.s and 15mPa.s and 15mPa.s formulation have the following components:

| | 6 mPa.s | 6/15 mPa.s | 15 mPa.s |
|---|---|---|---|
| Nifedipine | 3 | 3 | 3 |
| Pluronic® F-127 | 12 | 12 | 12 |
| HPMC | 5 | 30/30 | 60 |
| Lactose | 79 | 24 | 24 |
| Mg Stearate | 1 | 1 | 1 |

EXAMPLE 5

FIG. 6 is a graph that sets forth the release rates of tablets of a quick release and a sustained release formulation of nilvadipine-Pluronic® F-127 complex with different types of HPMC. These data may be utilized to select formulations to give desired release rates. The quick release and sustained release formulations are from Example 2.

FIG. 7 is a graph that illustrates the release rate of a 20 mg bilayer tablet that is made of 8mg of the quick release and 12 mg of the sustained release formulations shown on FIG. 6. These data show the result of the simultaneous administration of selected amounts of a quick release and a sustained release formulation in a bilayer tablet.

## Claims

### Claims for the following Contracting States : AT, BE, CH, DE, FR, GB IT, LI, NL, SE

**1.** A complex which comprises a 1,4-dihydropyridine of the general formula

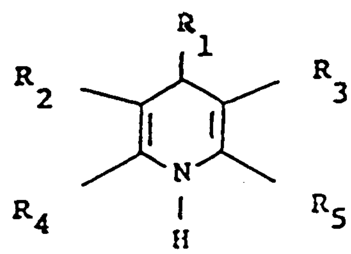

wherein $R_1$ is aryl;

$R_2$ and $R_3$ are the same or different and are ester groups or carboxy groups; and

$R_4$ and $R_5$ are hydrogen, cyano, $C_{1-8}$-alkyl, or substituted $C_{1-8}$-alkyl in which the substituent is cyano, hydroxy, acyloxy, hydroxyimino, hydrazono, $C_{1-8}$-alkoxyimino, hydroxy($C_{1-8}$)alkylimino, N'- or N',N'-di($C_{1-8}$)alkylamino-($C_{1-8}$)alkylimino, hydrazino, hydroxy($C_{1-8}$)alkylamino, N'- or N',N'-di($C_{1-8}$)-alkylamino($C_{1-8}$)alkylamino, a 5- or 6-membered saturated N-containing heterocyclic-1-yl which may have hydroxy, $C_{1-8}$-alkyl or hydroxy($C_{1-8}$)alkyl, or oxo wherein the thus formed carbonyl may be protected with a suitable protecting group, provided that, when one of $R_4$ and $R_5$ is hydrogen or $C_{1-8}$-alkyl, the other is always cyano or said substituted $C_{1-8}$-alkyl, and when $R_4$ and $R_5$ are not hydrogen or $C_{1-8}$-alkyl, both of them are a group selected from cyano and said substituted $C_{1-8}$-alkyl,

or $R_4$ is hydrogen or $C_{1-8}$-alkyl and $R_3$ and $R_5$ are combined to form a group of the formula

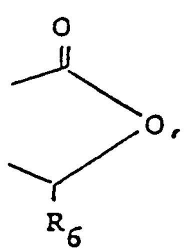 , 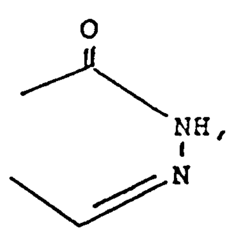 , 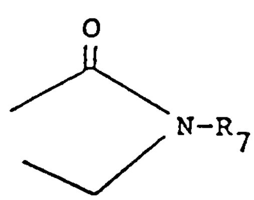 or 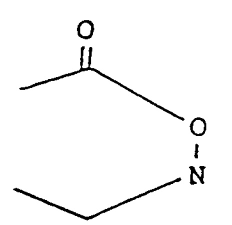

wherein $R_6$ is hydrogen or methyl and $R_7$ is 2-(N,N-diethyl amino)-ethyl or 2-hydroxyethyl, complexed with a block copolymer having the general structure

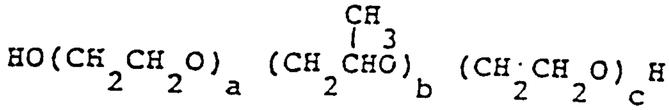

wherein a,b and c are selected to provide a molecular weight of from 1,000 to 16,000 and from 10% to 80% by weight of oxyethylene units.

**2.** A complex as defined in Claim 1 wherein the 1,4-dihydropyridine is nilvadipine, nitrendipine, nisoldipine, niludipine, nicardipine, nifedipine, felodipine or nimodipine.

**3.** A complex as defined in Claim 2 wherein the 1,4-dihydropyridine is nilvadipine.

**4.** A complex as defined in Claim 1 wherein the 1,4-dihydropyridine is nifedipine..

**5.** A pharmaceutical composition which comprises the complex of Claim 1 and a pharmaceutical excipient.

**6.** A pharmaceutical composition which comprises the complex of Claim 3.

**7.** A pharmaceutical unit dosage form as defined in Claim 6 wherein said pharmaceutical dosage form is a tablet and the tablet is a bilayer tablet which contains a quick release layer and a sustained release layer.

**8.** A sustained release dosage formulation which comprises one or more water soluble cellulose derivatives having a molecular weight which provides a viscosity in water of 9 to 30 mPa.s at 20 ° C as a 2% w/w aqueous solution and a 1,4-dihydropyridine according to claim 1 complexed with a polyoxypropylene-polyoxyethylene copolymer.

**9.** A quick release dosage formulation which comprises one or more water soluble cellulose derivatives having a molecular weight which provides a viscosity in water of 3 to 8 mPa.s a 20 ° C as a 2% solution in water and a 1,4-dihydropyridine according to claim 1 complexed with a polyoxypropylene polyoxyethylene copolymer.

**Claims for the following Contracting States : ES, GR**

**1.** A process for preparing a complex which comprises a 1,4-dihydropyridine of the general formula

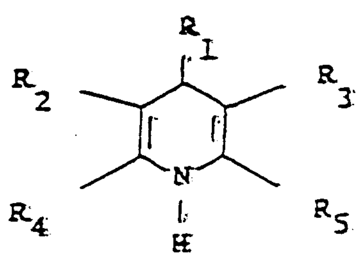

wherein $R_1$ is aryl;

$R_2$ and $R_3$ are the same or different and are ester groups or carboxy groups; and

$R_4$ and $R_5$ are hydrogen, cyano, $C_{1-8}$-alkyl, or substituted $C_{1-8}$-alkyl in which the substituent is cyano, hydroxy, acyloxy, hydroxyimino, hydrazono, $C_{1-8}$-alkoxyimino, hydroxy($C_{1-8}$)alkylimino, N'- or N',N'-di($C_{1-8}$)alkylamino-($C_{1-8}$)alkylimino, hydrazino, hydroxy($C_{1-8}$)alkylamino, N'- or N',N'-di($C_{1-8}$)-alkylamino($C_{1-8}$)alkylamino, a 5- or 6-membered saturated N-containing heterocyclic-1-yl which may have hydroxy, $C_{1-8}$-alkyl or hydroxy($C_{1-8}$)alkyl, or oxo wherein the thus formed carbonyl may be protected with a suitable protecting group, provided that, when one of $R_4$ and $R_5$ is hydrogen or $C_{1-8}$-alkyl, the other is always cyano or said substituted $C_{1-8}$-alkyl, and when $R_4$ and $R_5$ are not hydrogen

or $C_{1-8}$-alkyl, both of them are a group selected from cyano and said substituted $C_{1-8}$-alkyl, or $R_4$ is hydrogen or $C_{1-8}$-alkyl and $R_3$ and $R_5$ are combined to form a group of the formula

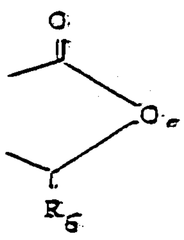 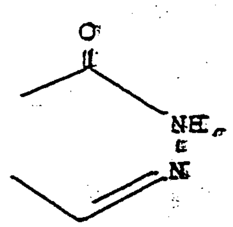 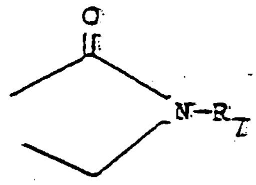 or 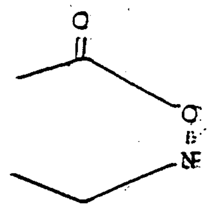

wherein $R_6$ is hydrogen or methyl and $R_7$ is 2-(N,N-diethylamino)-ethyl or 2-hydroxyethyl, complexed with a block copolymer having the general structure

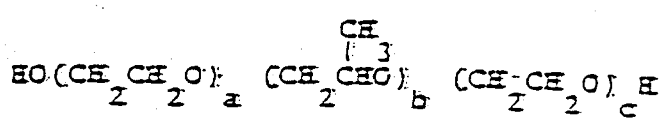

wherein a,b and c are selected to provide a molecular weight of from 1,000 to 16,000 and from 10% to 80% by weight of oxyethylene units,
said process comprising contacting the 1,4-dihydropyridine with the block copolymer in the presence of a suitable solvent at a temperature of 25 to 90 °C under atmospheric pressure or super-atmospheric pressure at a ratio of 1,4-dihydropyridine : block copolymer of from 1 : 1 to 1 : 10 (w/w).

**2.** A process as defined in claim 1 wherein the 1,4-dihydropyridine is nilvadipine, nitrendipine, nisoldipine, niludipine, nicardipine, nifedipine, felodipine or nimodipine.

**3.** A process as defined in Claim 2 wherein the 1,4-dihydropyridine is nilvadipine.

**4.** A process as defined in Claim 1 wherein the 1,4-dihydropyridine is nifedipine.

**5.** A pharmaceutical composition which comprises the complex of claim 1 and a pharmaceutical excipient.

**6.** A pharmaceutical composition which comprises the complex of claim 3.

**7.** A pharmaceutical unit dosage form as defined in claim 6 wherein said pharmaceutical unit dosage form is a tablet and the tablet is a bilayer tablet which contains a quick release layer and a sustained release layer.

**8.** A sustained release dosage formulation which comprises one or more water soluble cellulose derivatives having a molecular weight which provides a viscosity in water of 9 to 30 mPa.s at 20 °C as a 2% w/w aqueous solution and a 1,4-dihydropyridine according to claim 1 complexed with a polyoxypropylene-polyoxyethylene copolymer.

**9.** A quick release dosage formulation which comprises one or more water soluble cellulose derivatives having a molecular weight which provides a viscosity in water of 3 to 8 mPa.s at 20 °C as a 2% solution in water and a 1,4-dihydropyridine according to claim 1 complexed with a polyoxypropylene-polyoxyethylene copolymer.

## Revendications

**Revendications pour les Etats contractants suivants : AT, BE, CH, DE, FR, GB, IT, LI, NL, SE**

**1.** Complexe qui comporte une 1,4-dihydropyridine de formule générale:

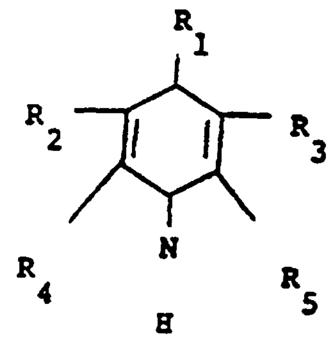

dans laquelle $R_1$ présente un groupe aryle,
$R_2$ et $R_3$, qui peuvent être identiques ou différents, représentent un groupe ester ou un groupe carboxy et
$R_4$ et $R_5$ représentent chacun un atome d'hydrogène un groupe cyano, alkyle ($C_{1-8}$) ou alkyle ($C_{1-8}$) substitué dans lequel le substituant est un groupe cyano, hydroxy, acyloxy, hydroxyimino, hydrazono, alkoxyimino ($C_{1-8}$), hydroxyalkylamino ($C_{1-8}$), N'- ou N',N'-di-alkylamino($C_{1-8}$)-alkylamino($C_{1-8}$), hydrazino, hydroxyalkylamino($C_{1-8}$), N'- ou N',N'-di-alkylamino($C_{1-8}$)-alkylamino($C_{1-8}$) ou un groupe hétérocyclique-1-yle azoté, saturé et composé de 5 ou 6 membres qui peut comporter un groupe hydroxy, alkyle($C_{1-8}$), hydroxyalkyle($C_{1-8}$) ou oxo dans lequel le carbonyle ainsi formé peut être protégé par un groupement protecteur approprié, pourvu que, si l'un des groupes $R_4$ et $R_5$ représente un atome d'hydrogène ou un groupe alkyle($C_{1-8}$), l'autre représente toujours un groupe cyano ou ledit alkyle($C_{1-8}$) substitué, et que, si $R_4$ et $R_5$ ne représentent ni un atome d'hydrogène ni un groupe alkyle($C_{1-8}$), ils représentent tous les deux soit un groupe cyano, soit ledit groupe alkyle ($C_{1-8}$) substitué,
ou bien $R_4$ représente un atome d'hydrogène ou un groupe alkyle ($C_{1-8}$) et $R_3$ et $R_5$ sont associés pour former un groupe correspondant à l'une des formules suivantes:

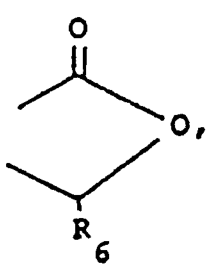
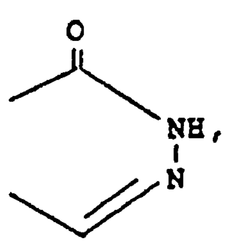
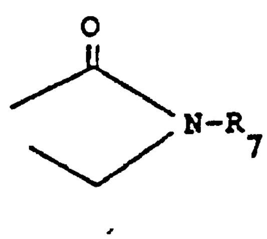
ou
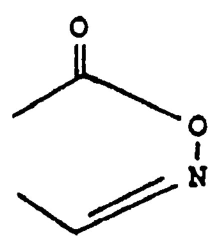

dans lesquelles $R_6$ représente un atome d'hydrogène ou un groupe méthyle et $R_7$ un groupe 2-(N,N-diéthylamino)-éthyle ou 2-hydroxyéthyle,
formant un complexe avec un copolymère séquencé de structure générale:

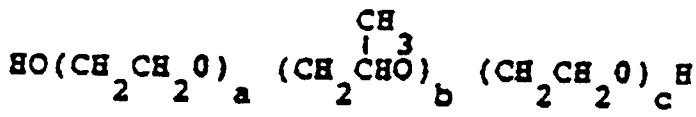

où a, b et c sont choisis de telle manière que le poids moléculaire moyen soit de 1 000 à 16 000 et de 10 à 80% en poids d'unités d'oxyéthylène.

2. Complexe selon la revendication 1 dans lequel la 1,4-dihydropyridine est la nilvadipine, la nitrendipine, la nisoldipine, la niludipine, la nicardipine, la nifédipine la félodipine ou la nomodipine.

3. Complexe selon la revendication 2 dans lequel la 1,4-dihydropyridine est la nilvadipine.

4. Complexe selon la revendication 1 dans lequel la 1,4-dihydropyridine est la nifédipine.

5. Composition pharmaceutique qui comporte le complexe de la revendication 1 et un excipient pharmaceutique.

6. Composition pharmaceutique qui comporte le complexe de la revendication 3.

7. Dose unitaire d'administration pharmaceutique selon la revendication 6 dans laquelle ladite forme d'administration pharmaceutique est un comprimé et ledit comprimé est un comprimé bicouche contenant une couche de délivrance rapide et une couche de délivrance retardée.

8. Formulation à délivrance retardée qui comporte un ou plusieurs dérivés hydrosolubles de la cellulose dont le poids moléculaire permet d'obtenir une viscosité dans l'eau de 9 à 30 mPa.s à 20 ° C en solution aqueuse à 2% p/p. et une 1,4-dihydropyridine selon la revendication 1 complexée avec un copolymère de polyoxypropylène-polyoxyéthylène.

9. Formulation à délivrance rapide qui comporte un ou plusieurs dérivés hydrosolubles de la cellulose dont le poids moléculaire permet d'obtenir une viscosité dans l'eau de 3 à 8 mPa.s à 20 ° C en solution aqueuse à 2% p/p. et une 1,4-dihydropyridine selon la revendication 1 complexée avec un copolymère de polyoxypropylène-polyoxyéthylène.

**Revendications pour les Etats contractants suivants : ES, GR**

1. Procédé de préparation d'un complexe qui comporte une 1,4-dihydropyridine de formule générale:

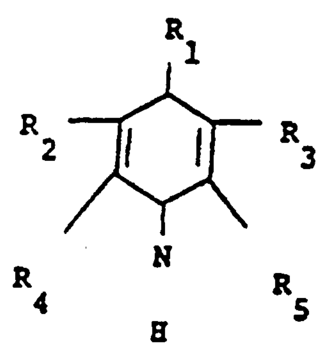

dans laquelle $R_1$ représente un groupe aryle,
$R_2$ et $R_3$, qui peuvent être identiques ou différents, représentent un groupe ester ou un groupe carboxy et
$R_4$ et $R_5$ représentent chacun un atome d'hydrogène, un groupe cyano, alkyle ($C_{1-8}$) ou alkyle ($C_{1-8}$) substitué dans lequel le substituant est un groupe cyano, hydroxy, acyloxy, hydroxyimino, hydrazono, alkoxyimino ($C_{1-8}$), hydroxyalkylamino($C_{1-8}$), N'- ou N',N'-di-alkylamino($C_{1-8}$)-alkylamino($C_{1-8}$), hydra-

zino, hydroxyalkylamino($C_{1-8}$), N'- ou N',N'-di-alkylamino($C_{1-8}$)-alkylamino($C_{1-8}$) ou un groupe hétérocyclique-1-yle azoté, saturé et composé de 5 ou 6 membres qui peut comporter un groupe hydroxy, alkyle($C_{1-8}$), hydroxyalkyle($C_{1-8}$) ou oxo dans lequel le carbonyle ainsi formé peut être protégé par un groupement protecteur approprié, pourvu que, si l'un des groupes $R_4$ et $R_5$ représente un atome d'hydrogène ou un groupe alkyle($C_{1-8}$), l'autre représente toujours un groupe cyano ou ledit alkyle($C_{1-8}$) substitué, et que, si $R_4$ et $R_5$ ne représentent ni un atome d'hydrogène ni un groupe alkyle ($C_{1-8}$), ils représentent tous les deux soit un groupe cyano, soit ledit groupe alkyle ($C_{1-8}$) substitué,
ou bien $R_4$ représente un atome d'hydrogène ou un groupe alkyle ($C_{1-8}$) et $E_3$ et $R_5$ sont associés pour former un groupe correspondant à l'une des formules suivantes:

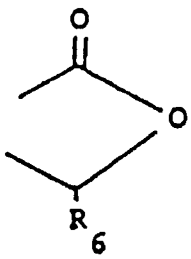 , 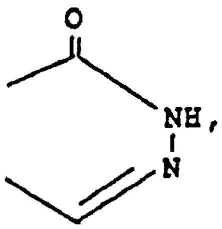 , 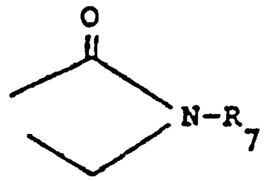 ou 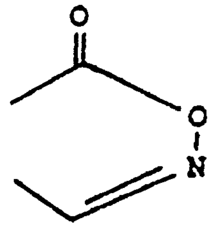

dans lesquelles $R_6$ représente un atome d'hydrogène ou un groupe méthyle et $R_7$ un groupe 2-(N,N-diéthylamino)-éthyle ou 2-hydroxyéthyle,
formant un complexe avec un copolymère séquencé de structure générale:

$$HO(CH_2CH_2O)_a\ (CH_2\overset{CH_3}{\overset{|}{C}}HO)_b\ (CH_2CH_2O)_cH$$

où a, b et c sont choisis de telle manière que le poids moléculaire moyen soit de 1 000 à 16 000 et de 10 à 80% en poids d'unités d'oxyéthylène.

**2.** Procédé selon la revendication 1 dans lequel la 1,4-dihydropyridine est la nilvadipine, la nitrendipine, la nisoldipine, la niludipine, la nicardipine, la nifédipine la félodipine ou la nomodipine.

**3.** Procédé selon la revendication 2 dans lequel la 1,4-dihydropyridine est la nilvadipine.

**4.** Procédé selon la revendication 1 dans lequel la 1,4-dihydropyridine est la nifédipine.

**5.** Composition pharmaceutique qui comporte le complexe de la revendication 1 et un excipient pharmaceutique.

**6.** Composition pharmaceutique qui comporte le complexe de la revendication 3.

**7.** Dose unitaire d'administration pharmaceutique selon la revendication 6 dans laquelle ladite forme d'administration pharmaceutique est un comprimé et ledit comprimé est un comprimé bicouche contenant une couche de délivrance rapide et une couche de délivrance retardée.

**8.** Formulation à délivrance retardée qui comporte un ou plusieurs dérivés hydrosolubles de la cellulose dont le poids moléculaire permet d'obtenir une viscosité dans l'eau de 9 à 30 mPa.s à 20°C en solution aqueuse à 2% p/p. et une 1,4-dihydropyridine selon la revendication 1 complexée avec un copolymère de polyoxypropylène-polyoxyéthylène.

9. Formulation à délivrance rapide qui comporte un ou plusieurs dérivés hydrosolubles de la cellulose dont le poids moléculaire permet d'obtenir une viscosité dans l'eau de 3 à 8 mPa.s à 20 ° C en solution aqueuse à 2% p/p. et une 1,4-dihydropyridine selon la revendication 1 complexée avec un copolymère de polyoxypropylène-polyoxyéthylène.

## Patentansprüche

### Patentansprüche für folgende Vertragsstaaten : AT, BE, CH, DE, FR, GB, IT, LI, NL, SE

1. Komplex, umfassend ein 1,4-Dihydropyridin der allgemeinen Formel

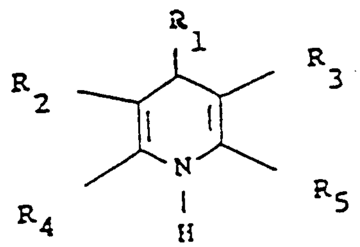

wobei $R_1$ für Aryl steht;

$R_2$ und $R_3$ gleich oder verschieden sind und Estergruppen oder Carboxylgruppen sind; und

$R_4$ und $R_5$ für Wassrstoff, Cyano, $C_{1-8}$-Alkyl oder substituiertes $C_{1-8}$-Alkyl stehen, wobei der Substituent Cyano, Hydroxy, Acyloxy, Hydroxyimino, Hydrazono, $C_{1-8}$-Alkoxyimino, Hydroxy($C_{1-8}$)-alkylimino, N'- oder N',N'-Di($C_{1-8}$)alkylamino-($C_{1-8}$)alkylimino, Hydrazino, Hydroxy($C_{1-8}$)alkylamino, N'- oder N',N'-Di($C_{1-8}$)alkylamino(($C_{1-8}$)alkylamino, ein 5- oder 6-gliedriger, gesättigter N-haltiger Heterocyclus-1-yl, der Hydroxy, $C_{1-8}$-Alkyl oder Hydroxy($C_{1-8}$)alkyl aufweisen kann, oder Oxo ist, wobei das so gebildete Carbonyl geschützt sein kann mit einer geeigneten Schutzgruppe, mit der Maßgabe, daß dann, wenn eines von $R_4$ und $R_5$ Wasserstoff oder $C_{1-8}$-Alkyl ist, das andere immer Cyano oder das erwähnte substituierte $C_{1-8}$-Alkyl ist, und daß dann, wenn $R_4$ und $R_5$ nicht Wasserstoff oder $C_{1-8}$-Alkyl sind, beide von ihnen für eine Gruppe stehen, ausgewählt aus Cyano und dem erwähnten substituierten $C_{1-8}$-Alkyl,

oder $R_4$ für Wasserstoff oder $C_{1-8}$-Alkyl steht und $R_3$ und $R_5$ kombiniert sind unter Bildung einer Gruppe der Formel

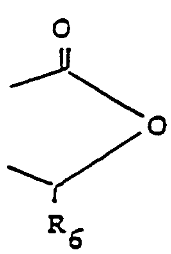 , 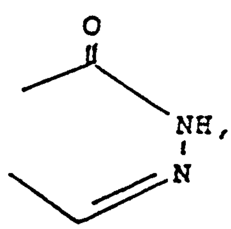 , 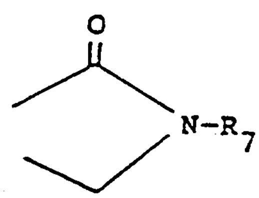 oder 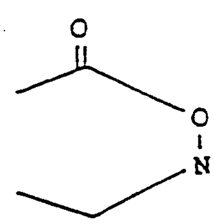

wobei $R_6$ für Wasserstoff oder Methyl steht und $R_7$ für 2-(N,N-Diethylamino)ethyl oder 2-Hydroxyethyl steht,

komplexiert mit einem Blockcopolymeren mit der allgemeinen Struktur

$$\overset{\qquad\qquad\qquad\qquad\qquad CH_3}{HO(CH_2CH_2O)_a\ (CH_2CHO)_b\ (CH_2CH_2O)_cH}$$

wobei a, b und c so ausgewählt sind, daß ein Molekulargewicht von 1 000 bis 16 000 erhalten wird und von 10 bis 80 Gew.% Oxyethyleneinheiten vorhanden sind.

2. Komplex gemäß Anspruch 1, wobei das 1,4-Dihydropyridin Nilvadipin, Nitrendipin, Nisoldipin, Niludipin, Nicardipin, Nifedipin, Felodipin oder Nimodipin.

3. Komplex gemäß Anspruch 2, wobei das 1,4-Dihydropyridin Nilvadipin ist.

4. Komplex gemäß Anspruch 1, wobei das 1,4-Dihydropyridin Nifedipin ist.

5. Pharmazeutische Zusammensetzung, umfassend den Komplex von Anspruch 1 und ein pharmazeutisches Streckmittel.

6. Pharmazeutische Zusammensetzung, welche den Komplex von Anspruch 3 umfaßt.

7. Pharmazeutische Einheitsdosisform gemäß Anspruch 6, wobei die pharmazeutische Dosisform eine Tablette ist und die Tablette eine Zweischichtentablette ist, welche eine Schicht mit rascher Freigabe und eine Schicht mit verzögerter Freigabe enthält.

8. Dosisformulierung mit verzögerter Freigabe, umfassend ein oder mehrere wasserlösliche Cellulosederivate, welche ein derartiges Molekulargewicht haben, daß sie als eine 2%-ige Gew./Gew. wässrige Lösung zu einer Viskosität in Wasser von 9 bis 30 mPa.s bei 20 ° C führen, und ein 1,4-Dihydropyridin gemäß Anspruch 1, komplexiert mit einem Polyoxypropylenpolyoxyethylen-Copolymeren.

9. Dosisformulierung mit rascher Freigabe, umfassend ein oder mehrere wasserlösliche Cellulosederivate, welche ein derartiges Molekulargewicht aufweisen, daß sie als eine 2%-ige Lösung in Wasser zu einer Viskosität in Wasser von 3 bis 8 mPa.s bei 20 ° C führen, und ein 1,4-Dihydropyridin gemäß Anspruch 1, komplexiert mit einem Polyoxypropylenpolyoxyethylen-Copolymeren.

**Patentansprüche für folgende Vertragsstaaten : ES, GR**

1. Verfahren zur Herstellung eines Komplexes, umfassend ein 1,4-Dihydropyridin der allgemeinen Formel

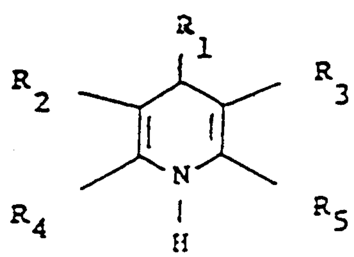

wobei $R_1$ für Aryl steht;

$R_2$ und $R_3$ gleich oder verschieden sind und Estergruppen oder Carboxylgruppen sind; und

$R_4$ und $R_5$ für Wassrstoff, Cyano, $C_{1-8}$-Alkyl oder substituiertes $C_{1-8}$-Alkyl stehen, wobei der Substituent Cyano, Hydroxy, Acyloxy, Hydroxyimino, Hydrazono, $C_{1-8}$-Alkoxyimino, Hydroxy($C_{1-8}$)-alkylimino, N'- oder N',N'-Di($C_{1-8}$)alkylamino-($C_{1-8}$)alkylimino, Hydrazino, Hydroxy($C_{1-8}$)alkylamino, N'- oder N',N'-Di($C_{1-8}$)alkylamino($C_{1-8}$)alkylamino, ein 5- oder 6-gliedriger, gesättigter N-haltiger Heterozyclus-1-yl, der Hydroxy, $C_{1-8}$-Alkyl oder Hydroxy($C_{1-8}$)alkyl aufweisen kann, oder Oxo ist, wobei das so gebildete Carbonyl geschützt sein kann mit einer geeigneten Schutzgruppe, mit der Maßgabe, daß dann, wenn eines von $R_4$ und $R_5$ Wasserstoff oder $C_{1-8}$-Alkyl ist, das andere immer Cyano oder das erwähnte substituierte $C_{1-8}$-Alkyl ist, und daß dann, wenn $R_4$ und $R_5$ nicht Wasserstoff oder $C_{1-8}$-Alkyl sind, beide von ihnen für eine Gruppe stehen, ausgewählt aus Cyano und dem erwähnten substituierten $C_{1-8}$-Alkyl,

oder $R_4$ für Wasserstoff oder $C_{1-8}$-Alkyl steht und $R_3$ und $R_5$ kombiniert sind unter Bildung einer Gruppe der Formel

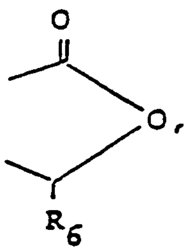, 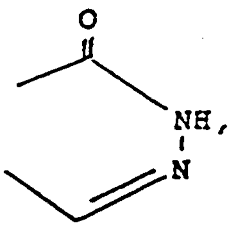, 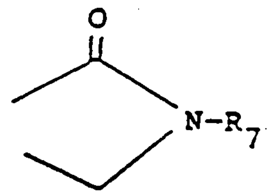 oder 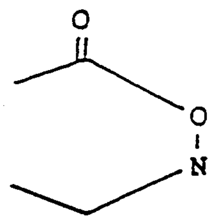

wobei $R_6$ für Wasserstoff oder Methyl steht und $R_7$ für 2-(N,N-Diethylamino)ethyl oder 2-Hydroxyethyl steht,
komplexiert mit einem Blockcopolymeren mit der allgemeinen Struktur

$$HO(CH_2CH_2O)_a\ (CH_2\overset{\overset{\large CH_3}{|}}{C}HO)_b\ (CH_2CH_2O)_cH$$

wobei a, b und c so ausgewählt sind, daß ein Molekulargewicht von 1 000 bis 16 000 erhalten wird und von 10 bis 80 Gew.% Oxyethyleneinheiten vorhanden sind, umfassend die Kontaktierung des 1,4-Dihydropyridins mit dem Blockcopolymeren in Anwesenheit eines geeigneten Lösungsmittels bei einer Temperatur von 25 bis 90 ° C unter atmosphärischem Druck oder überatmosphärischem Druck bei einem Verhältnis von 1,4-Dihydropyrydin : Blockcopolymeres von 1 : 1 bis 1 : 10 (Gew./Gew.).

**2.** Verfahren gemäß Anspruch 1, wobei das 1,4-Dihydropyridin Nilvadipin, Nitrendipin, Nisoldipin, Niludipin, Nicardipin, Nifedipin, Felodipin oder Nimodipin.

**3.** Verfahren gemäß Anspruch 2, wobei das 1,4-Dihydropyridin Nilvadipin ist.

**4.** Verfahren gemäß Anspruch 1, wobei das 1,4-Dihydropyridin Nifedipin ist.

**5.** Pharmazeutische Zusammensetzung, umfassend den Komplex von Anspruch 1 und ein pharmazeutisches Streckmittel.

**6.** Pharmazeutische Zusammensetzung, welche den Komplex von Anspruch 3 umfaßt.

**7.** Pharmazeutische Einheitsdosisform gemäß Anspruch 6, wobei die pharmazeutische Dosisform eine Tablette ist und die Tablette eine Zweischichtentablette ist, welche eine Schicht mit rascher Freigabe und eine Schicht mit verzögerter Freigabe enthält.

**8.** Dosisformulierung mit verzögerter Freigabe, umfassend ein oder mehrere wasserlösliche Cellulosederivate, welche ein derartiges Molekulargewicht haben, daß sie als eine 2%-ige Gew./Gew. wässrige Lösung zu einer Viskosität in Wasser von 9 bis 30 mPa.s bei 20 ° C führen, und ein 1,4-Dihydropyridin gemäß Anspruch 1, komplexiert mit einem Polyoxypropylenpolyoxyethylen-Copolymeren.

**9.** Dosisformulierung mit rascher Freigabe, umfassend ein oder mehrere wasserlösliche Cellulosederivate, welche ein derartiges Molekulargewicht aufweisen, daß sie als eine 2%-ige Lösung in Wasser zu einer Viskosität in Wasser von 3 bis 8 mPa.s bei 20 ° C führen, und ein 1,4-Dihydropyridin gemäß Anspruch 1, komplexiert mit einem Polyoxypropylenpolyoxyethylen-Copolymeren.

BEHAVIOR OF NILVADIPINE - PLURONIC COMPLEX IN BIOLOGICAL SYSTEMS

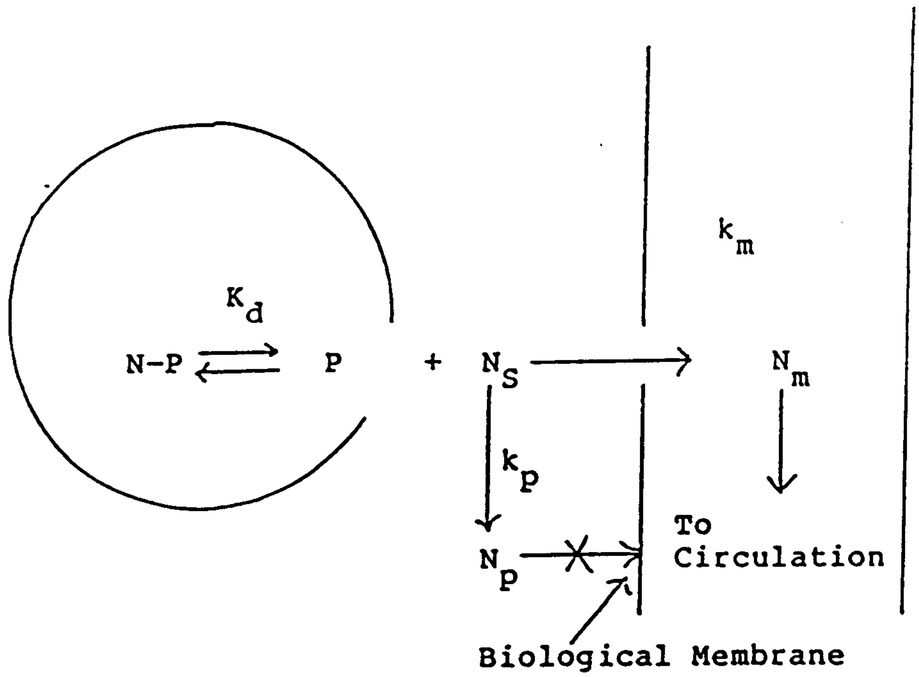


N-P = Highly soluble Nilvadipine-pluronic complex

$N_p$ = Precipitated low solubility form of Nilvadipine

$N_s$ = Nilvadipine in a solution state

$N_m$ = Nilvadipine in biological membrane

$K_d$ = Dissociation constant of Nilvadipine - polymer complex

$k_p$ = Rate constant for $N_s$ to $N_p$

$k_m$ = Rate constant of $N_s$ bioabsorption

FIGURE 1

DISSOLUTION PROFILES OF 10mg NIFEDIPINE TABLETS
(NIFEDIPINE COMPLEX, (SUSTAINED RELEASE): PHYS
MIX, CONTROL)

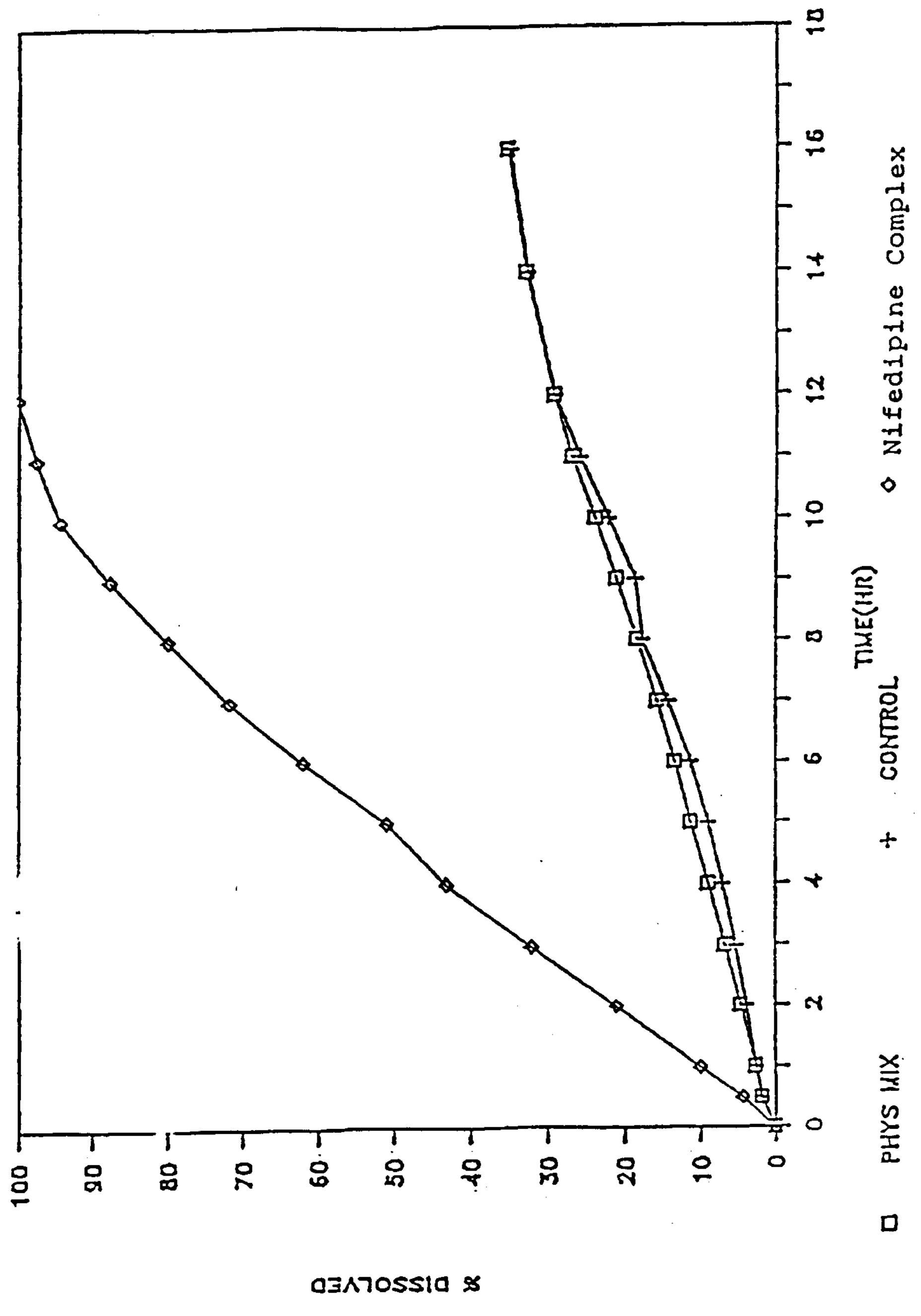


FIGURE 2

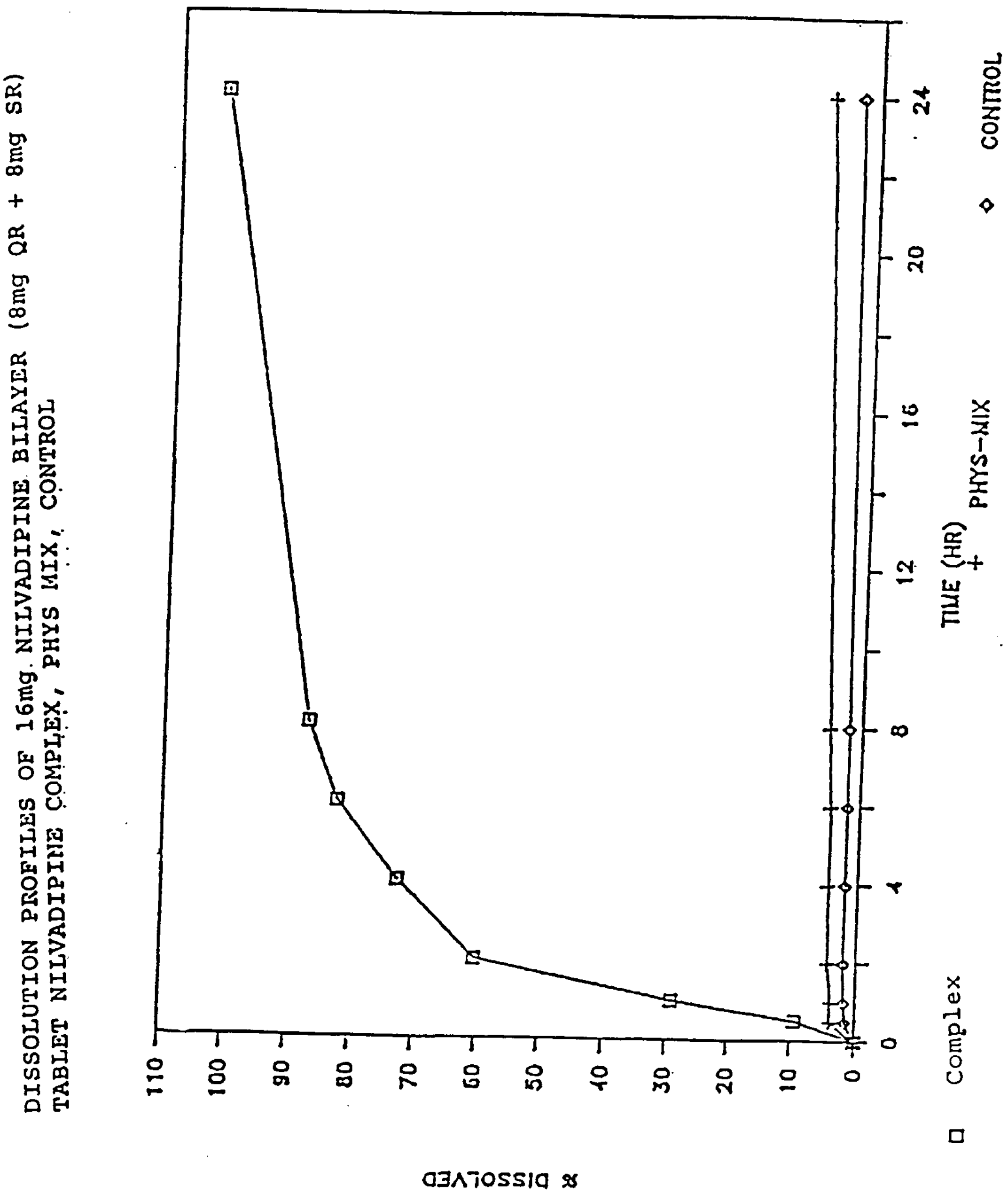
DISSOLUTION PROFILES OF 16mg NILVADIPINE BILAYER (8mg QR + 8mg SR)
TABLET NILVADIPINE COMPLEX, PHYS MIX, CONTROL
% DISSOLVED
110
100
90
80
70
60
50
40
30
20
10
0
0
4
8
12
16
20
24
TIME (HR)
Complex
PHYS-MIX
CONTROL

FIGURE 3

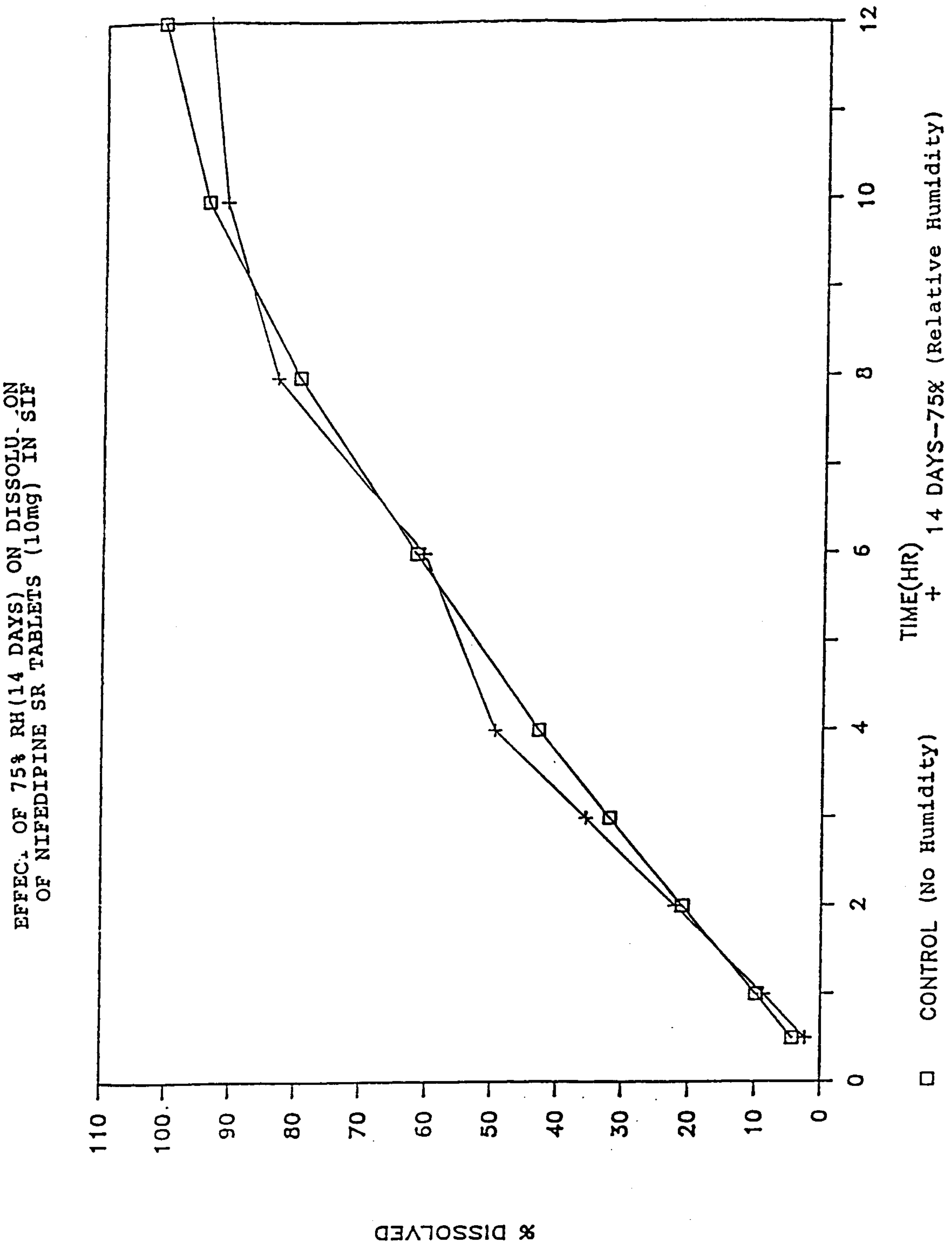
EFFECT OF 75% RH(14 DAYS) ON DISSOLUTION
OF NIFEDIPINE SR TABLETS (10mg) IN SIF
% DISSOLVED
110
100
90
80
70
60
50
40
30
20
10
0
0
2
4
6
8
10
12
TIME(HR)
CONTROL (No Humidity)
14 DAYS-75% (Relative Humidity)

FIGURE 4

DISSOLUTION PROFILES OF 10mg NIFEDIPINE TABLETS AS A FUNCTION OF CELLULOSE POLYMER BLEND

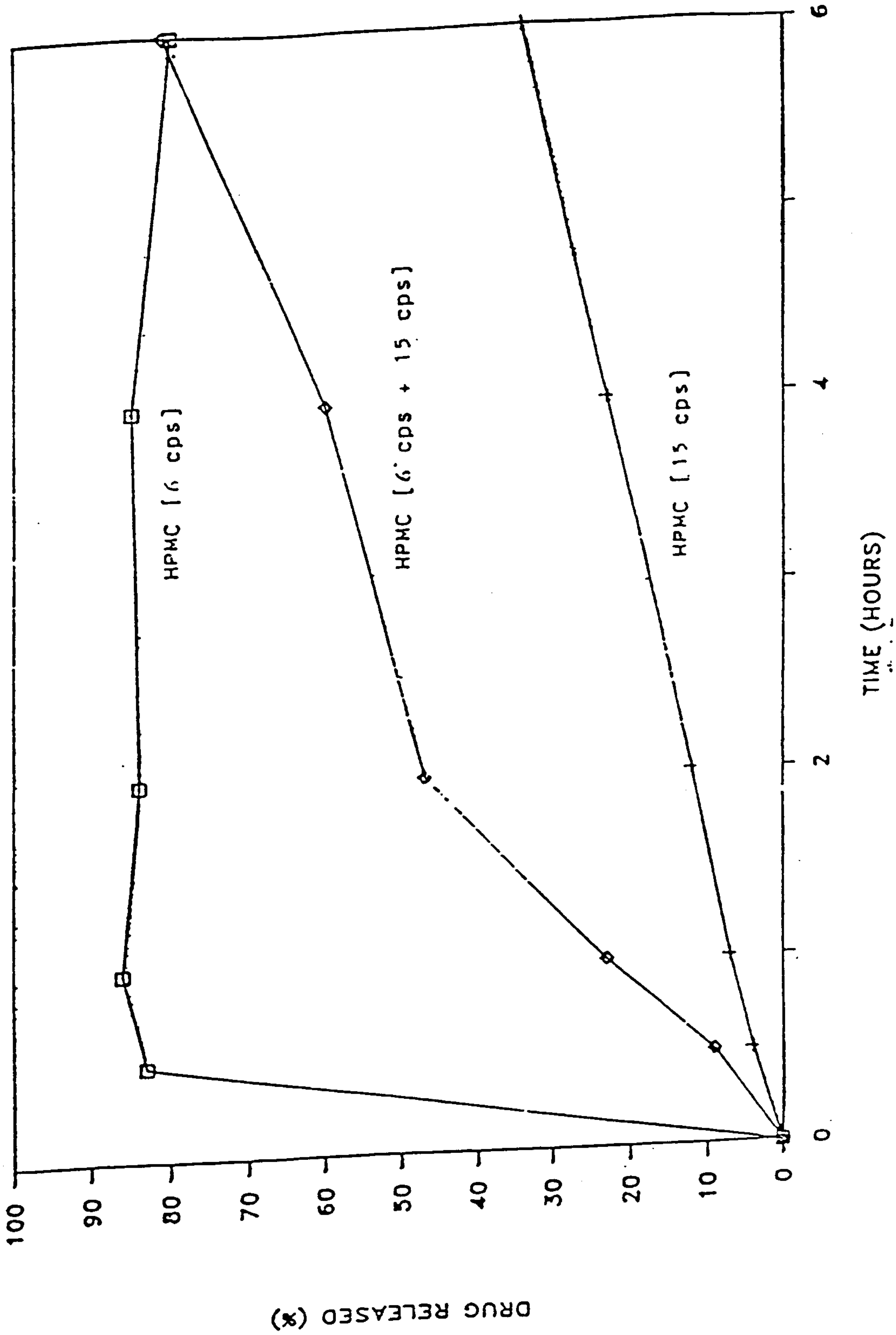


FIGURE 5

DISSOLUTION AT 37°C IN SIF CONTAINING 0.4% TWEEN® 20 OF NILVADIPINE QUICK RELEASE AND SUSTAINED RELEASE TABLETS

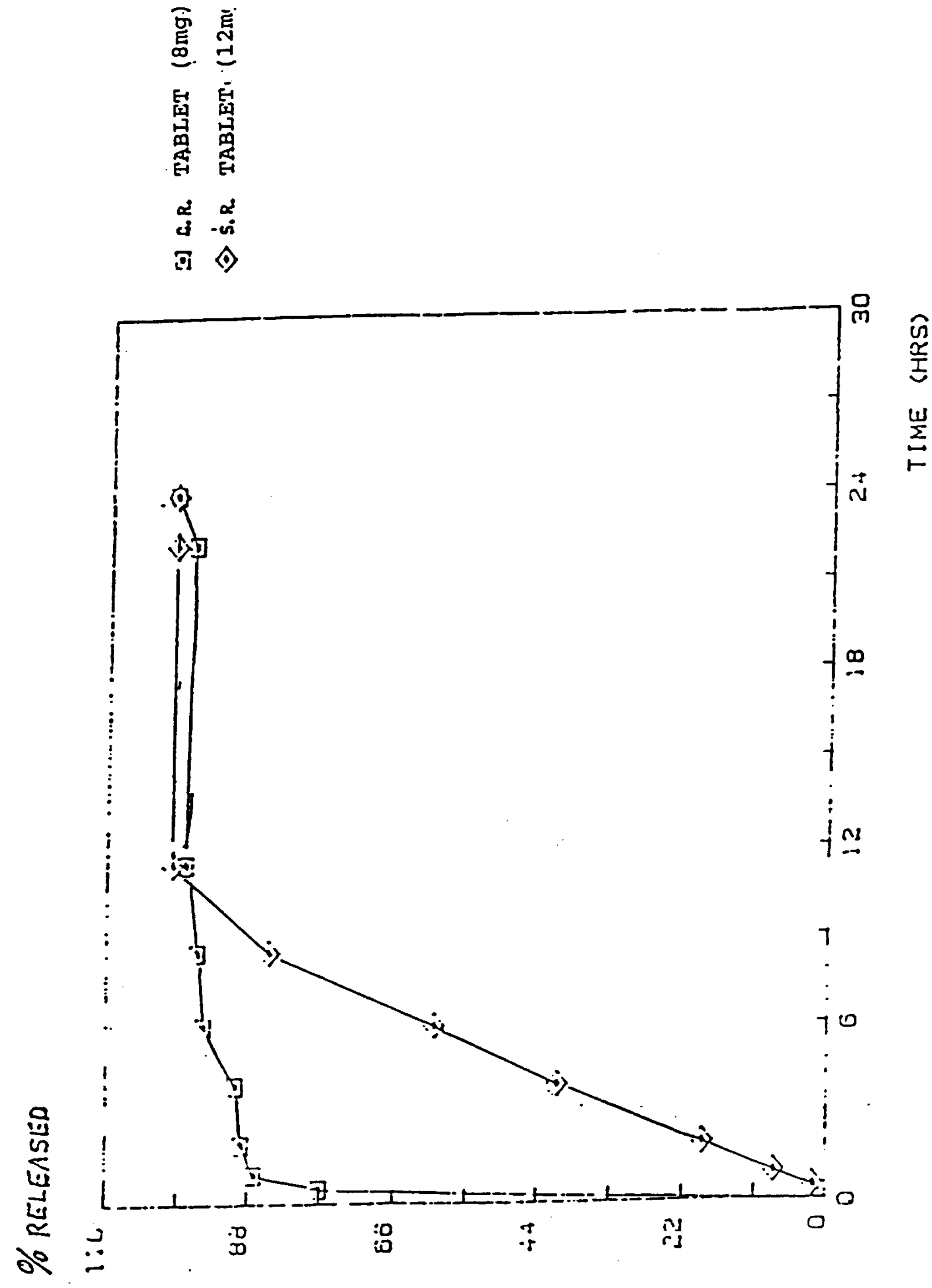


FIGURE 6

DISSOLUTION OF 20 mg BILAYER NILVADIPINE (8 mg Q.R. + 12 mg S.R.)
AT 37°C IN SIMULATED INTESTINAL FLUID CONTAINING 0.4% TWEEN® 20.

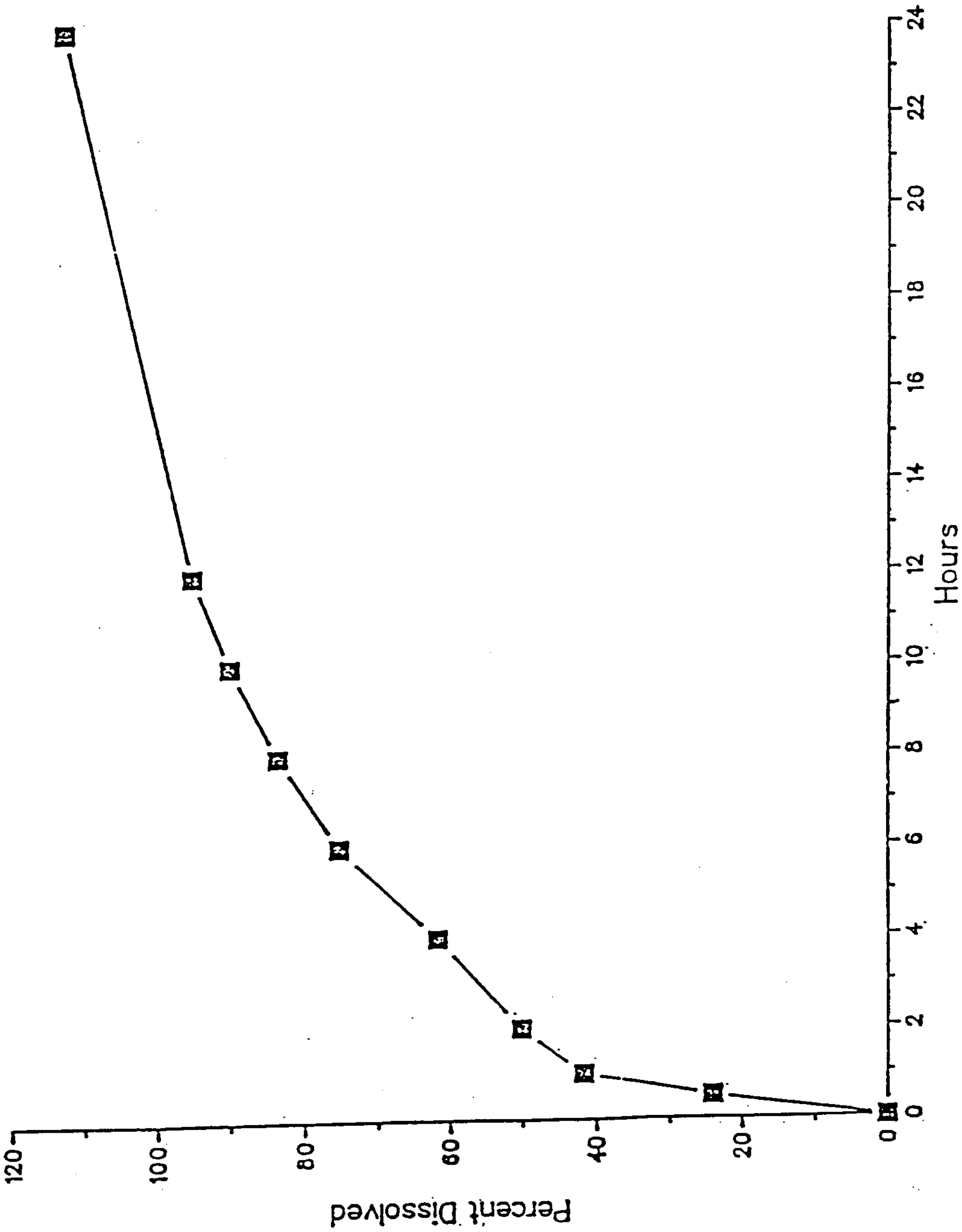


FIGURE 7